# EUROPEAN PATENT APPLICATION

(11) **EP 0 745 891 A2**
(43) Date of publication of application: **04.12.1996**
(21) Application number: 96202357.8
(22) Date of filing: 17.06.1993
(51) Int. Cl.: G03B 42/04

(54) **X-ray apparatus and arrangement for position indication**

(30) Priority: 25.06.1992 EP 92201870
(62) Divisional of application: 93201738.7
(71) Applicant: PHILIPS ELECTRONICS N.V., 5621 BA Eindhoven (NL)
(72) Inventor: Hoeks, Antonius Johannes Ludovicus Maria, 5656 AA Eindhoven (NL)
(74) Representative: Cohen, Julius Simon

(57) **Abstract**

An x-ray examination apparatus comprises an arrangement (5) for remotely controlled positioning a marker (9a-d) to be imaged on an x-ray sensitive element in an x-ray exposure region. The arrangement comprises a U-shaped flat frame (10,11) for carrying markers (9a-d) to be imaged at opposite ends of the frame (10,11). The frame (10,11) is moveable with respect to the x-ray exposure region such that, depending on the position of the frame, either one or none of said markers (9a-d) is brought in the x-ray exposure region.

## Description

The invention relates to an x-ray examination apparatus comprising an arrangement for remotely controlled positioning a marker to be imaged on an x-ray sensitive element in an x-ray exposure region,

An x-ray examination apparatus of said kind is known from the German *Patentschrift* **DE 32 28 577**.

For performing appropriate medical treatment on a basis of a diagnosis obtained by using an x-ray image, it is desirable that information on orientation and/or position of a patient during x-ray examination is recorded on x-ray film together with the x-ray image. This is achieved by imaging relevant markers, e.g. lead-letters, which are at option placed in the x-ray beam. In the known x-ray examination apparatus, information on the position and the orientation of a patient during examination is provided in x-ray images by imaging lead-letters simultaneously with imaging the patient. In the known x-ray examination apparatus, a disc containing a plurality of different lead-letters, each of them corresponding to one of a plurality of patient positions and/or orientations, is rotatably attached to an x-ray shutter. Implicitly it is clear, that the known x-ray examination apparatus comprises an x-ray detector consisting of a film holder containing x-ray sensitive film-material. A relevant choice of letters to be imaged on x-ray images can be made by suitable rotation of the disc. The disc is driven by an arrangement of cables, pulleys, a winch and a motor; said arrangement being placed in an extension of the film holder along a longitudinal direction of the patient table.

The known x-ray apparatus is only able to place a marker in a fixed position in the x-ray exposure region.

An object of the invention is to provide an x-ray examination apparatus is more flexible as regards placing a marker in the x-ray exposure region.

This object is achieved by an x-ray examination apparatus according to the invention which is characterised in that the arrangement comprises a U-shaped flat frame for carrying markers to be imaged at opposite ends of the frame, the frame being moveable with respect to the x-ray exposure region such that, depending on the position of the frame, either one or none of said markers is brought in the x-ray exposure region.

For forming an x-ray image an x-ray sensitive element such as an x-ray film or an x-ray image intensifier is positioned in the x-ray exposure region. Having markers, notably lead-letters, attached to separate frame parts, rather than to any other moveable parts of an x-ray examination apparatus, such as e.g. to x-ray shutters that are moveable in correspondence to film cassette formats, renders possible to position lead-letters in the x-ray exposure region, independently of positions of any other moveable parts of an x-ray examination apparatus. Therefore, lead-letters can be positioned more freely, so as to image a lead-letter in an x-ray image without a relevant lead-letter compromising visibility of relevant information contained in the x-ray image. Furthermore, the frame is formed such that at most only a single marker can be imaged. Hence, it is avoided that two markers are simultaneously imaged, which would cause confusion.

A preferred embodiment of x-ray examination apparatus according to the invention is characterised in that the U-shaped frame has two opposite side elements and an elongate base element,the base element connecting said opposite side elements, each side element carrying a marker to be imaged at the one end which is remote from the elongate base element and the frame being moveable in a longitudinal direction of the elongate base element

The length of the elongate base element is suitably chosen such that when one marker enters the x-ray exposure region, the other marker on the frame is outside of the x-ray exposure region. Further the length of the elongate base element is such that at a stand-by position of the frame none of the markers on the frame is within the x-ray exposure region.

A preferred embodiment of x-ray examination apparatus according to the invention is characterised in that the arrangement for remotely controlled positioning a marker comprises at least two said U-shaped frames.

The more frames are provided, the more markers can be accommodated. In particular it is advantageous to provide a first frame having a set of markers for anterior-posterior x-ray exposures and another frame having a set of markers for posterior-anterior exposures.

These and other aspects of the invention are elucidated with respect to the embodiments discussed hereinafter and with reference to the accompanying drawing wherein
Figure 1 shows a side elevation of an x-ray examination apparatus in which the invention is employed
Figure 2 shows a plan view of an arrangement for remotely-controlled positioning a marker in the x-ray exposure region.

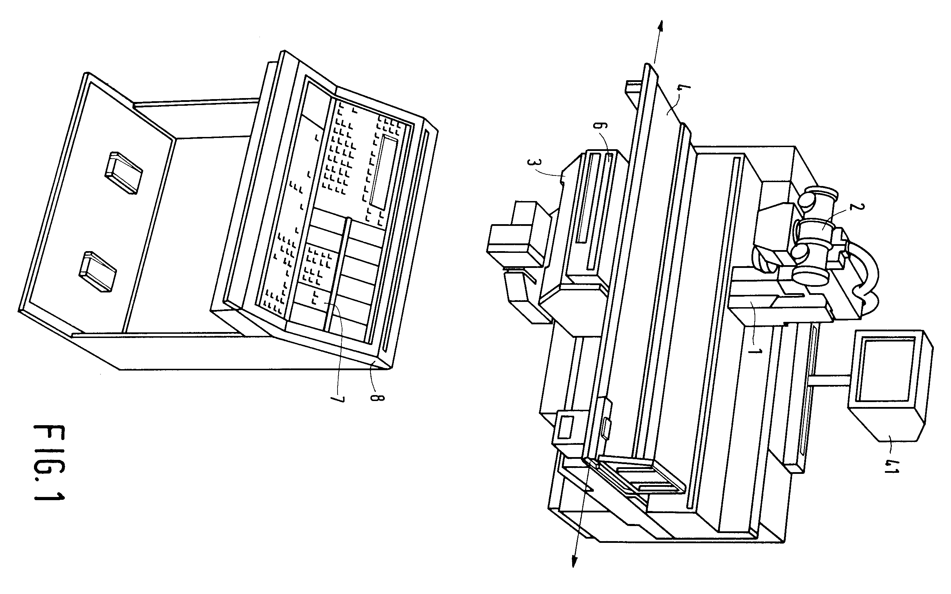

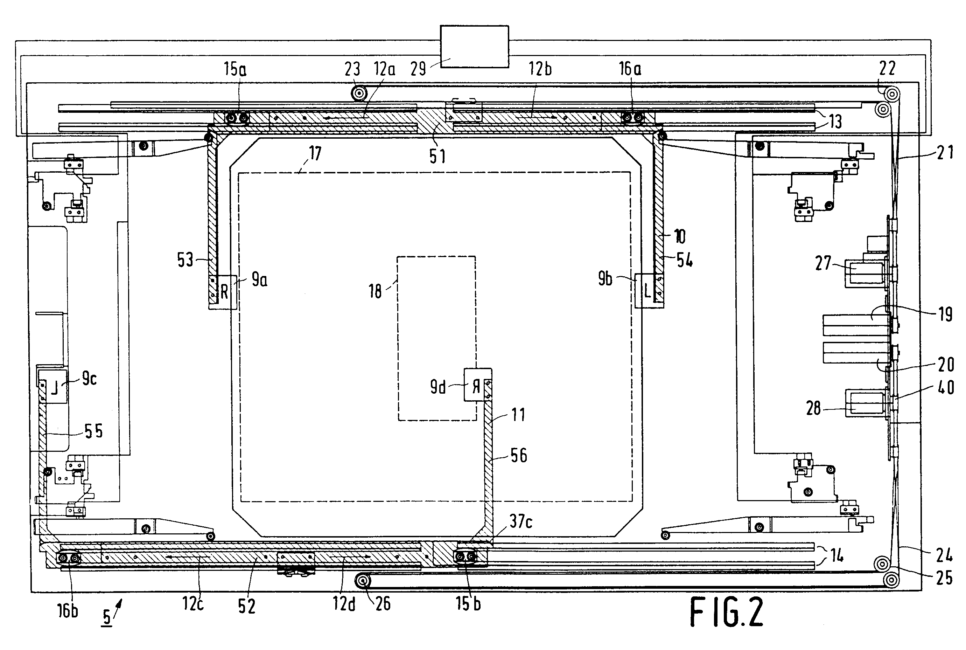

An x-ray examination apparatus as shown in Figure 1 comprises a carrier 1 supporting an x-ray source 2 for generating an x-ray beam. An x-ray detector 3, having the form of a serial film changer is facing the x-ray source. A table 4 for supporting a patient is situated between the x-ray source and the x-ray detector. An arrangement 5 for remote-controlled position indication on x-ray images produced by an x-ray examination apparatus in accordance with the invention is incorporated in the serial film changer. The arrangement 5 is not visible in Figure 1, but will be further discussed in the sequel with reference to Figure 2. For producing x-ray images on hard-copy, notably x-ray film, a film cassettes is inserted into a slot 6 of the serial film changer. The image plane is then formed by the film that is incorporated in the film cassette. The side of the serial film changer comprising the slot will be designated hereinafter as the front side of the serial film changer; correspondingly, the side of the serial film changer opposite said front side will be referred to hereinafter as the rear side of the serial film changer. As an alternative to employing a film cassette for producing x-ray images, an x-ray image intensifier can be used as an x-ray detector. Image information generated by the x-ray image intensifier can be displayed on a monitor 41 or can be supplied to further image processing means. Whenever an x-ray image intensifier is employed for x-ray imaging, the input screen of said image intensifier constitutes the image plane wherein an x-ray image is formed. The image format displayed on the monitor depends on various adjustments of the image intensifier and e.g. also on an x-ray beam collimator which is incorporated in the x-ray source 1. The table supporting the patient is moveable *inter alia* as indicated by the arrows. The x-ray examination apparatus can be operated by means of a control panel 7 on a control desk 8.

Figure 2 shows a plan view of an arrangement 5 for remote-controlled patient position and/or orientation indication on x-ray images in accordance with the invention. Position indication is performed by imaging appropriate markers consisting of lead-letters together with the imaging of a patient. A set of four lead-letters 9a-d are provided. A first pair consisting of 9a and 9b is attached to a first moveable frame 10 and a second pair consisting of 9c and 9d is attached to a second moveable frame 11. The first frame 10 and the second frame 11 are moveable in the directions indicated by arrows 12a and 12b, and by arrows 12c and 12d, respectively. The arrangement 5 for remote controlled position indication is incorporated in the serial film changer in such a way that the plane in which the markers 9a-d are moveable is substantially parallel to a surface of the patient table 4, and motors 19,20 for driving the frames are placed at the rear end of the serial film changer. To facilitate motion of the frames sets of guide rails 13 and 14 are provided and the frame part 9 is provided with guide means 15a and 16a and frame part 10 is provided with guide means 15b and 16b. The frame part 10 is shown in Figure 2 positioned in a standby position. In this standby position neither lead-letter 9a, nor lead-letter 9b is imaged for any film cassette format that is suitable for use in an x-ray examination apparatus in accordance with the invention. A contour indicating an outer edge of a film cassette of a largest format suitable for use in an x-ray examination apparatus in accordance with the invention is shown in Figure 2 by way of the dashed contour 17. A contour indicating the outer edge of a film cassette of a smallest format suitable for use in an x-ray examination apparatus in accordance with the invention is shown in Figure 2 by way of the dashed contour 18. Frame 11 is shown in Figure 2 positioned in an exposure position. In this exposure position lead-letter 9d is imaged on an x-ray image made employing a film cassette of the smallest format suitable for use in an x-ray examination apparatus in accordance with the invention. Each of the frames 10,11 comprises an elongate base member 51,52 which connects opposite side members 53,54,55,56. The size of the elongate base member is such that at most one of the markers on the same frame can be placed in the exposure region. The length of the elongate base member is such that when one marker enters the region of the largest film format 17, the marker on the opposite side of the same frame has left that region. As the length of the elongate base member allows at most one marker of the same frame in the region of the largest film format, also there is at most one marker of the same frame in the region of smaller film formats

The motors 19,20 are provided as drive means for displacing frames and lead-letter attached to them. The motion of frames and lead-letters attached to them is driven by motors. A first motor 19 is provided for driving the motion of frame 10 along guide rails 13, by means of a belt 21 and systems of pulleys 22 and 23. A second motor 20 is provided for driving the motion of frame 11 along guide rails 14, by means of a belt 24 and systems of pulleys 25 and 26. For controlling the motion of frame 10 control means comprising a first potentiometer 27 is provided that is driven by the motor 19 and the belt 21. For controlling the motion of frame 11 control means comprising a second potentiometer 28 is provided that is driven by the motor 20 and the belt 24. Signals in correspondence with a required number of revolutions of motor 19 and of motor 20, respectively, the required number of revolutions of said motors being in correspondence with a required distance over which any of the frame parts 10 or 11 is required to move, are provided by a micro-processor unit 29; said signals being supplied by the micro-processor unit to a relevant potentiometer.

An arrangement as presented in Figure 2 having four lead-letters is particularly advantageous, because such an arrangement provides for indicating 'left' and 'right' in an anterior-to-posterior exposure by way of imaging an 'L' or an 'R', shown here attached to frame 10 as lead-letters 9a, and 9b, respectively, and such an arrangement also provided for indicating 'left' and 'right' in a posterior-to-anterior exposure by way of imaging an 'inverted L' or and 'inverted R', shown here attached to frame 11 as lead-letters 9c and 9c, respectively.

## Claims

1. An x-ray examination apparatus comprising
- an arrangement (5) for remotely controlled positioning a marker (9a-d) to be imaged on an x-ray sensitive element in an x-ray exposure region,
characterised in that
- the arrangement comprises a U-shaped flat frame (10,11) for carrying markers (9a-d) to be imaged at opposite ends of the frame,
- the frame being moveable with respect to the x-ray exposure region
- such that, depending on the position of the frame, either one or none of said markers is brought in the x-ray exposure region.

2. An x-ray examination apparatus as claimed in Claim 1, characterised in that
- the U-shaped frame (10,11) has two opposite side elements (53,54,55,56) and an elongate base element (51,52),
- the base element (51,52) connecting said opposite side elements (53,54,55,56),
- each side element (53,54,55,56) carrying a marker (9a-d) to be imaged at the one end which is remote from the elongate base element (51,52) and
- the frame (10,11) being moveable in a longitudinal direction of the elongate base element (51,52).

3. An x-ray examination apparatus as claimed in Claim 1 or 2, characterised in that
- the arrangement (5) for remotely controlled positioning a marker (9a-d) comprises at least two said U-shaped frames (10,11).
